(19) Europäisches Patentamt
European Patent Office
Office européen des brevets

(11) **EP 3 082 715 B1**

(12) **EUROPEAN PATENT SPECIFICATION**

(45) Date of publication and mention
of the grant of the patent:
**14.02.2018 Bulletin 2018/07**

(51) Int Cl.:
*A61K 8/25* *(2006.01)*    *A61K 8/26* *(2006.01)*
*A61Q 11/00* *(2006.01)*    *A61K 8/19* *(2006.01)*
*A61K 8/24* *(2006.01)*    *A61K 8/73* *(2006.01)*

(21) Application number: **13815341.6**

(22) Date of filing: **16.12.2013**

(86) International application number:
**PCT/US2013/075306**

(87) International publication number:
**WO 2015/094153 (25.06.2015 Gazette 2015/25)**

(54) **ORAL CARE COMPOSITIONS COMPRISING CALCIUM CARBONATE AND TALC**

MUNDPFLEGE-VERBINDUNG ENTHALTEND KALZIUM KARBONATE UND TALK

COMPOSITION POUR HYGIENE BUCCALE CONTENANT DU CARBONATE DE CALCIUM ET DU TALC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.10.2016 Bulletin 2016/43**

(73) Proprietor: **Colgate-Palmolive Company New York, NY 10022 (US)**

(72) Inventors:
• **D'AMBROGIO, Robert**
 **Princeton, New Jersey 08540 (US)**
• **POTANIN, Andrei**
 **Hillsborough, New Jersey 08844 (US)**

• **PAN, Guisheng**
 **Philadelphia, Pennsylvania 19114 (US)**
• **LIN, Nora**
 **Basking Ridge, New Jersey 07920 (US)**

(74) Representative: **Wibbelmann, Jobst et al Wuesthoff & Wuesthoff Patentanwälte PartG mbB Schweigerstrasse 2 81541 München (DE)**

(56) References cited:
**WO-A1-96/32090    WO-A1-99/33437
DE-A1- 2 239 197    US-A- 2 995 521**

**Description**

**BACKGROUND**

It world be desirable to provide more cost-effective calcium carbonate-based anti-cavity toothpastes than those currently available, which exhibit consumer-acceptable characteristics such as acceptable viscosity and good chemical and physical stabilities.

**BRIEF SUMMARY**

In one aspect, the present invention provides an oral care composition comprising, by total weight of the composition: (a) 15 to 35 weight % calcium carbonate; and (b) 10 to 20 weight % talc: wherein the total concentration of talc and calcium carbonate in the composition is from greater than 35 weight % to 42 weight %, by total weight of the composition; and wherein the calcium carbonate comprises natural calcium carbonate or precipitated calcium carbonate.

Optionally, the oral care composition comprises from 20 to 30 weight % calcium carbonate, based on the total weight of the composition.

Optionally, the oral care composition further comprises from 0.5 to 2 weight % cellulose ether thickening agent, based on the total weight of the composition. Further optionally, the oral care composition comprises from 1 to 1.5 weight % cellulose ether thickening agent, based on the total weight of the composition.

Optionally, the oral care composition further comprises an inorganic thickening agent. Further optionally, the inorganic thickening agent comprises thickening silica. Still further optionally, the oral care composition comprises from 1 to 6 weight % thickening silica, based on the total weight of the composition. Yet further optionally, the oral care composition comprises from 1 to 2 weight % thickening silica, based on the total weight of the composition.

Optionally, the inorganic thickening agent comprises a clay thickening agent. Further optionally, the oral care composition comprises from 0.5 to 2 weight % clay thickening agent, based on the total weight of the composition.

In another aspect, the present invention also provides an oral care composition comprising, by total weight of the composition: (a) 15 to 30 weight % calcium carbonate; (b) 5 to 20 weight % talc; and (c) an additional structuring agent; wherein the total concentration of talc and calcium carbonate is 35 weight % or less, and wherein the additional structuring agent comprises at least one of: (i) a cellulose ether thickening agent; and (ii) an inorganic thickening agent selected from thickening silica, a clay thickening agent, and mixtures thereof.

Optionally, the oral care composition comprises 18 to 22 weight % calcium carbonate, based on the total weight of the composition.

Optionally, the oral care composition comprises 7 to 15 weight % talc, based on the total weight of the composition.

Optionally, the oral care composition comprises from 0.5 to 2 weight % cellulose ether thickening agent, based on the total weight of the composition. Further optionally, the oral care composition comprises from 1.6 to 2 weight % cellulose ether thickening agent, based on the total weight of the composition.

Optionally, the oral care composition comprises from 1 to 6 weight % thickening silica, based on the total weight of the composition. Further optionally, the oral care composition comprises from 2 to 5 weight % thickening silica, based on the total weight of the composition.

Optionally, the oral care composition comprises from 0.5 to 2 weight % clay thickening agent, based on the total weight of the composition. Further optionally, the oral care composition comprises from 1 to 2 weight % clay thickening agent, based on the total weight of the composition.

Optionally, in either of the above aspects, the talc comprises natural talc, synthetic talc, modified talc, or mixtures thereof. Further optionally, the talc is natural talc. Optionally, the talc comprises macrocrystalline talc. Optionally, the talc has a purity level of at least 89 % by weight. Optionally, the mean particle size of the talc is within the range of from 8 to 28 $\mu$m.

Optionally, in either of the above aspects, the cellulose ether thickening agent comprises carboxymethylcellulose; sodium carboxymethyl cellulose; hydroxymethylcellulose; hydroxyethylcellulose or derivatives thereof; hydroxypropylcellulose or derivatives thereof; hydroxypropylmethylcellulose or derivatives thereof; or mixtures thereof. Further optionally, the cellulose ether thickening agent is sodium carboxymethylcellulose.

Optionally, in either of the above aspects, the clay thickening agent is a natural or synthetic clay comprising sodium, calcium, magnesium, lithium or potassium cations, or mixtures thereof. Optionally, the clay thickening agent is a smectite, bentonite, montmorillonite, hectorite or attipulgite clay. Further optionally, the clay thickening agent is magnesium aluminum silicate.

Optionally, in either of the above aspects, the clay thickening agent is a synthetic layered silicate. Further optionally, the clay thickening agent is magnesium lithium silicate.

Optionally, in either of the above aspects, the oral care compositions further comprise a preservative. Further optionally, the preservative is selected from benzyl alcohol and parabens. Optionally, the preservative is present in an amount of from 0.1 to 1 weight %, based on the total weight of the composition.

Optionally, in either of the above aspects, the oral care compositions further comprise a humectant. Optionally, the humectant is selected from sorbitol, glycerin, xylitol, polyethylene glycol, propylene glycol, and combinations thereof. Further optionally, the humectant is sorbitol. Further optionally, the humectant is glycerin. Optionally, the humectant is present in an amount of from 5 to 25 weight %, based on the total weight of the composition. Further optionally, the humectant is present in an amount of from 10 to 15 weight %, based on the total weight of the composition.

Optionally, in either of the above aspects, the compositions have a pH of from 9.2 to 10.2. Optionally, the compositions further comprise a buffer system, the buffer system being: (a) a combination of sodium silicate and tetrasodium pyrophosphate; (b) a combination of sodium hydroxide, sodium bicarbonate and tetrasodium pyrophosphate; or (c) a combination of sodium bicarbonate and sodium carbonate. Optionally, the buffer system is 0.25 to 0.75 weight % sodium silicate and 0.25 to 0.75 weight % tetrasodium pyrophosphate, based on the total weight of the composition. Optionally, the buffer system is 0.03 to 0.5 weight % sodium hydroxide, 0.25 to 0.75 weight % sodium bicarbonate and 0.25 to 1.5 weight % tetrasodium pyrophosphate, based on the total weight of the composition. Optionally, the buffer system is 0.05 to 0.5 weight % sodium bicarbonate and 0.2 to 0.6 weight % sodium carbonate, based on the total weight of the composition.

Optionally, in either of the above aspects, the composition is a toothpaste, a tooth gel, or a combination thereof.

Optionally, in either of the above aspects, the viscosity of the composition is from 100,000 to 1,000,000 cps as measured at 25°C at 1 rpm using a Brookfield Viscometer Model HADV-II + Pro and a V74 spindle.

Optionally, in either of the above aspects, the composition has a static yield stress of at least 50 Pa as measured at 25°C using a Brookfield Viscometer Model HADV-II + Pro and a V74 spindle.

In another aspect, the present invention also provides for use, in an oral care composition, of a combination of talc and calcium carbonate to improve flavor release.

Optionally, the calcium carbonate is present in the oral care composition in an amount of from 15 to 35 weight %, and the talc is present in the oral care composition in an amount of from 5 to 25 weight %, based on the total weight of the composition. Further optionally, the total concentration of talc and calcium carbonate in the oral care composition is greater than 35 weight %, based on the total weight of the composition.

Optionally, the calcium carbonate is present in the oral care composition in an amount of from 15 to 30 weight %, and the talc is present in an amount of from 5 to 20 weight %, based on the total weight of the composition. Further optionally, the total concentration of talc and calcium carbonate in the oral care composition is 35 weight % or less, based on the total weight of the composition, and wherein the composition comprises an additional structuring agent which comprises at least one of: (i) a cellulose ether thickening agent; and (ii) an inorganic thickening agent selected from thickening silica, a clay thickening agent, and mixtures thereof. Optionally, the oral care composition comprises from 0.5 to 2 weight % cellulose ether thickening agent, optionally from 1.6 to 2 weight % cellulose ether thickening agent, based on the total weight of the composition. Optionally, the oral care composition comprises from 1 to 6 weight % thickening silica, optionally from 2 to 5 weight % thickening silica, based on the total weight of the composition. Optionally, the oral care composition comprises from 0.5 to 2 weight % clay thickening agent, optionally from 1 to 2 weight % clay thickening agent, based on the total weight of the composition.

Further areas of applicability of the present invention will become apparent from the detailed description provided hereinafter.

## DETAILED DESCRIPTION

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present inventors have found that oral care compositions with acceptable cleaning efficacy, good chemical and physical stability and desirable consumer-relevant properties (such as desirable viscosity and yield stress) can be obtained by formulating the compositions with specific amounts of calcium carbonate and talc. The present inventors have found that replacing a portion of the calcium carbonate with talc maintains acceptable cleaning ability and physical stability. The compositions of the present invention are more cost-effective than previous "benchmark" formulations, and have good chemical and physical stability. The compositions of the present invention also have improved flavor release and exhibit reduced dry-out as compared to the previous "Benchmark" formulations.

Accordingly, in a first aspect, the present invention provides oral care composition comprising, by total weight of the composition: (a) 15 to 35 weight % calcium carbonate; and (b) 5 to 25 weight % talc; wherein the total concentration of talc and calcium carbonate in the composition is greater than 35 weight %.

In some embodiments, the total concentration of talc and calcium carbonate in the oral care composition is from greater than 35 weight % to 55 weight %, from greater than 35 weight % to 50 weight %, from greater than 35 weight % to 45 weight %, from greater than 35 weight % to 42 weight %, or from greater than 35 weight % to 40 weight %, by total weight of the composition.

In some embodiments, the oral care composition comprises from 16 to 34 weight %; from 17 to 33 weight %; from 18 to 32 weight %; from 19 to 31 weight %; from 20 to 30 weight %; about 20 weight %; or about 30 weight % calcium carbonate, based on the total weight of the composition.

In some embodiments, the calcium carbonate comprises natural calcium carbonate (NCC). In some embodiments, the calcium carbonate comprises precipitated calcium carbonate (PCC). In some embodiments, the calcium carbonate is natural calcium carbonate (PCC). In some embodiments, the calcium carbonate is precipitated calcium carbonate (PCC).

In some embodiments, the oral care composition comprises from 6 to 24 weight %; from 7 to 23 weight %; from 8 to 22 weight %; from 9 to 21 weight %; from 10 to 20 weight %, about 10 weight %; about 20 weight %; from 12 to 18 weight %; from 14 to 16 weight %; or about 15 weight % talc, based on the total weight of the composition.

Talc is a hydrated magnesium silicate ($Mg_3Si_4O_{10}(OH)_2$), formed by the hydrothermal actions and regional metamorphism of magnesium rich rocks like dolomite, chlorite, etc. Its theoretical chemical composition is expressed as 31.7% by weight MgO, 63.5 by weight % $SiO_2$ and 4.8 by weight % $H_2O$. It is a phyllosilicate having a structural unit containing three sheets, with an octahedrally coordinated magnesium hydroxide group (brucite) sandwiched between two layers of tetrahedrally-linked silica layers.

The size of an individual talc platelet (which is a few thousand elementary sheets), can vary from approximately one micron (1 $\mu$m) to over one hundred microns (100 $\mu$m) depending on the deposit. It is this individual platelet size that determines talc's lamellarity. Highly lamellar talc (macrocrystalline talc) has large individual platelets, whereas compact talc (microcrystalline talc) has much smaller, compact platelets. The elementary sheets, stacked on top of one another, are very weakly held together by van der Waals forces. Thus, the platelets slide apart at the slightest touch, giving talc its characteristic softness. It is the softest mineral and listed as 1 on the Mohs hardness scale. Natural talc of USP (United States Pharmacopoeia) grade tends to be low in cost and readily available in various global deposits.

In some embodiments, the talc comprises natural talc, synthetic talc, modified talc, or mixtures thereof. In some embodiments, the talc is natural talc. An example of a preferred talc is natural talc of USP (United States Pharmacoepia) grade. In certain embodiments, the talc comprises macrocrystalline talc. In certain embodiments, the talc comprises microcrystalline talc. In some embodiments, the talc is a combination of macrocrystalline and microcrystalline talc. In some embodiments, the talc has a purity level of at least 89 % by weight; at least 90 % by weight; at least 91 % by weight; at least 92 % by weight; at least 93 % by weight; at least 94 % by weight; at least 95 % by weight; at least 96 % by weight; at least 97 % by weight; at least 98 % by weight; or at least 99 % by weight. In some embodiments, the mean particle size of the talc is within the range of from 8 to 28 $\mu$m; from 10 to 26 $\mu$m; from 15 to 25 $\mu$m; from 16 to 23 $\mu$m; or from 17 to to $\mu$m.

Impurities/other minerals may be present in the talc material in different proportions (depending on mine location and methods of separation and further purification). In some embodiments, the talc comprises a chlorite impurity level of 12 weight % or lower; 11 weight % or lower; 10 weight % or lower; 9 weight % or lower; 8 weight % or lower; 7 weight % or lower; 6 weight % or lower; 5 weight % or lower; 4 weight % or lower; 3 weight % or lower; 2 weight % or lower; or 1 weight % or lower.

In some embodiments, the talc comprises a quartz impurity level of 5 weight % or lower; 4 weight % or lower; 3 weight % or lower; 2 weight % or lower; 1 weight % or lower; or less than 1 weight %.

In some embodiments, the talc comprises a calcite impurity level of 5 weight % or lower; 4 weight % or lower; 3 weight % or lower; 2 weight % or lower; 1 weight % or lower; or less than 1 weight %.

In some embodiments, the talc comprises a dolomite impurity level of 5 weight % or lower; 4 weight % or lower; 3 weight % or lower; 2 weight % or lower; 1 weight % or lower; or less than 1 weight %.

In some embodiments, the oral care composition further comprises from 0.5 to 2 weight %; from 0.9 to 1.6 weight %; from 1 to 1.5 weight %; about 1 weight %; about 1.25 weight %; about 1.3 weight %; or about 1.5 weight % cellulose ether thickening agent, based on the total weight of the composition. The cellulose ether thickening agent may comprise carboxymethylcellulose (CMC); sodium carboxymethyl cellulose (NaCMC); hydroxymethylcellulose (HMC); hydroxyethylcellulose (HEC) or derivatives thereof; hydroxypropylcellulose (HPC) or derivatives thereof; hydroxypropylmethylcellulose (HPMC) or derivatives thereof; or mixtures thereof In some embodiments, the cellulose ether thickening agent is sodium carboxymethylcellulose. Typical commercial NaCMC options have a degree of substitution (DS) of from 0.7 to 1.2 (i.e. for every 10 anhydroglucose units, 7 to 12 hydroxy groups will be substituted with carboxymethyl groups). In general, CMC becomes more hydrophilic with increasing DS level, and the performance of the gum is modified with different DS. For the compositions of the present invention, NaCMC with degrees of substitution from 0.7 (Type 7) to 1.2 (Type 12) may be used. Particular examples of sodium carboxymethylcellulose which may be used in the present invention include NaCMC Type 7 (such as Gelycel® from Amtex Chemicals, LLC) and NaCMC Type 8 (such as CMC-TMS from Chongqing Lihong Fine Chemical Co. Ltd.).

In some embodiments, the oral care composition further comprises an inorganic thickening agent. In certain embodiments, the inorganic thickening agent comprises thickening silica. Examples of thickening silicas which may be used are Zeodent 165, Zeodent 163 and Zeodent 153 (from Tuber); Aerosil® 200 and Sident® 22S (from Evonik); and Sylodent® 15 and Perkasil® SM 660 (from W.R. Grace & Co.); and Tixocil 43B (From Rhodia). The oral care composition may comprise from 1 to 6 weight %; from 1 to 3 weight %; from 1 to 2.5 weight %; from 1 to 2 weight %; from 1.5 to 2 weight %; or about 2 weight % thickening silica, based on the total weight of the composition.

In some embodiments, the inorganic thickening agent comprises a clay thickening agent. The clay thickening agent may be present in an amount of from 0.5 to 2 weight %; from 0.75 to 1.5 weight %; from 0.75 to 1.25 weight %; or about 1 weight %, based on the total weight of the composition. The clay thickening agent may be a natural or synthetic clay comprising sodium, calcium, magnesium, lithium or potassium cations, or mixtures thereof. In some embodiments, the clay thickening agent is a smectite, bentonite, montmorillonite, hectorite (such as Bentone® EW, LT from Rheox) or attipulgite (such as Attasorb® or Pharmasorb® from Enlgelhard, Inc.) clay, or mixtures thereof. Smectite clays are a class of natural mineral clays which exhibit high swelling/gelling and absorption properties, and can help to provide desirable structuring and rheology characteristics. In certain embodiments, the clay thickening agent is magnesium aluminum silicate (such as Veegum® from R.T. Vanderbilt Holding Company, Inc.), which is a purified bentonite clay within the smectite class.

In some embodiments, the clay thickening agent is a synthetic layered silicate, for example magnesium lithium silicate (such as Laponite™ from Rockwood).

In some embodiments, the oral care composition does not contain thickening silica. In some embodiments, the oral care composition does not contain a clay thickening agent. In some embodiments, the oral care composition does not contain thickening silica or a clay thickening agent.

In a second aspect, the present invention also provides an oral care composition comprising, by total weight of the composition: (a) 15 to 30 weight % calcium carbonate; (b) 5 to 20 weight % talc; and (c) an additional structuring agent; wherein the total concentration of talc and calcium carbonate is 35 weight% or less, and wherein the additional structuring agent comprises at least one of: (i) a cellulose ether thickening agent; and (ii) an inorganic thickening agent selected from thickening silica, a clay thickening agent, and mixtures thereof.

Optionally, the oral care composition comprises 18 to 22 weight % calcium carbonate, based on the total weight of the composition. In some embodiments, the oral care composition comprises from 16 to 25 weight %; from 17 to 23 weight %; from 18 to 22 weight %; from 19 to 21 weight %; or about 20 weight % calcium carbonate, based on the total weight of the composition.

In some embodiments, the calcium carbonate comprises natural calcium carbonate (NCC). In some embodiments, the calcium carbonate comprises precipitated calcium carbonate (PCC). In some embodiments, the calcium carbonate is natural calcium carbonate (NCC). In some embodiments, the calcium carbonate is precipitated calcium carbonate (PCC).

In some embodiments, the oral care composition comprises from 6 to 18 weight %; from 7 to 15 weight %; from 8 to 12 weight %; from 9 to 10 weight %; or about 10 weight % talc, based on the total weight of the composition.

In some embodiments, the talc comprises natural talc, synthetic talc, modified talc, or mixtures thereof. In some embodiments, the talc is natural talc. An example of a preferred talc is natural talc of USP (United States Pharmacoepia) grade. In certain embodiments, the talc comprises macrocrystalline talc. In certain embodiments, the talc comprises microcrystalline talc. In some embodiments, the talc is a combination of macrocrystalline and microcrystalline talc. In some embodiments, the talc has a purity level of at least 89 % by weight; at least 90 % by weight; at least 91 % by weight; at least 92 % by weight; at least 93 % by weight; at least 94 % by weight; at least 95 % by weight; at least 96 % by weight; at least 97 % by weight; at least 98 % by weight; or at least 99 % by weight. In some embodiments, the mean particle size of the talc is within the range of from 8 to 28 μm; from 10 to 26 μm; from 15 to 25 μm; from 16 to 23 μm; or from 17 to 20 μm.

Impurities/other minerals may be present in the talc material in different proportions (depending on mine location and methods of separation and further purification). In some embodiments, the talc comprises a chlorite impurity level of 12 weight % or lower; 11 weight % or lower; 10 weight % or lower, 9 weight % or lower; 8 weight % or lower, 7 weight % or lower; 6 weight % or lower; 5 weight % or lower; 4 weight % or lower; 3 weight % or lower; 2 weight % or lower; or 1 weight % or lower.

In some embodiments, the talc comprises a quartz impurity level of 5 weight % or lower; 4 weight % or lower; 3 weight % or lower; 2 weight % or lower; 1 weight % or lower; or less than 1 weight %.

In some embodiments, the talc comprises a calcite impurity level of 5 weight % or lower; 4 weight % or lower; 3 weight % or lower; 2 weight % or lower; 1 weight % or lower; or less than 1 weight %.

In some embodiments, the talc comprises a dolomite impurity level of 5 weight % or lower; 4 weight % or lower; 3 weight % or lower; 2 weight % or lower; 1 weight % or lower; or less than 1 weight %.

In some embodiments, the oral care composition comprises from 0.5 to 2 weight %; from 1.6 to 2 weight %; from 1.6 to 1.8 weight %; 1.6 weight %; or 1.8 weight % cellulose ether thickening agent, based on the total weight of the composition. The cellulose ether thickening agent may comprises carboxymethylcellulose (CMC); sodium carboxymethyl cellulose (NaCMC); hydroxymethylcellulose (HMC); hydroxyethylcellulose (HEC) or derivatives thereof; hydroxypropylcellulose (HPC) or derivatives thereof; hydroxypropylmethylcellulose (HPMC) or derivatives thereof; or mixtures thereof. In some embodiments, the cellulose ether thickening agent is sodium carboxymethylcellulose. For the compositions of the present invention, NaCMC with degrees of substitution from 0.7 (Type 7) to 1.2 (Type 12) may be used. Particular examples of sodium carboxymethylcellulose which may be used in the present invention include NaCMC Type 7 (such as Gelycel® from Amtex Chemicals, LLC) and NaCMC Type 8 (such as CMC-TMS from Chongqing Lihong Fine Chemical Co. Ltd.).

In some embodiments, the oral care composition comprises from 1 to 6 weight %; from 2 to 5 weight %; from 4 to 5 weight %; about 5 weight %; about 4 weight %; or about 2 weight % thickening silica, based on the total weight of the composition. Examples of thickening silicas which may be used are Zeodent 165, Zeodent 163 and Zeodent 153 (from Huber); Aerosil® 200 and Sident® 22S (from Evonik); Sylodent® 15 and Perkasil® SM 660 (from W.R. Grace & Co.); and Tixocil 43B (From Rhodia).

In some embodiments, the oral care composition comprises from 0.5 to 2 weight %; from 1 to 2 weight %; or about 1 weight % clay thickening agent, based on the total weight of the composition. The clay thickening agent may be a natural or synthetic clay comprising sodium, calcium, magnesium, lithium or potassium cations, or mixtures thereof. In some embodiments, the clay thickening agent is a smectite, bentonite, montmorillonite, hectorite (such as Bentone® EW, LT from Rheox) or attipulgite (such as Attasorb® or Pharmasorb® from Enlgelhard, Inc.) clay, or mixtures thereof In certain embodiments, the clay thickening agent is magnesium aluminum silicate (such as Veegum® from R.T. Vanderbilt Holding Company, Inc.), which is a purified bentonite clay within the smectite class. In some embodiments, the clay thickening agent is a synthetic layered silicate, for example magnesium lithium silicate (such as Laponite™ from Rockwood).

In some embodiments, the oral care composition does not contain thickening silica. In some embodiments, the oral care composition does not contain a clay thickening agent. In some embodiments, the oral care composition does not contain thickening silica or a clay thickening agent.

In any of the embodiments of the two aspects of the invention, as discussed above, the oral care composition may further comprise a preservative agent, such as benzyl alcohol or parabens such as methylparaben and propylparaben. In some embodiments, the preservative is benzyl alcohol. The preservative agent may be present in the composition in an amount of from 0.1 to 1 weight %; 0.2 to 0.5 weight %; or about 0.3 weight %, based on the total weight of the composition.

In any of the above embodiments of the two aspects of the present invention as discussed above, the oral care composition may comprise at least 35 weight % water, based on the total weight of the composition. In some embodiments, the oral care composition comprises from 35 to 55 weight %; from 35 to 50 weight %; from 35 to 45 weight %; or from 38 to 45 weight % water, based on the total weight of the composition.

In some embodiments, the oral care compositions further comprise a humectant. In certain embodiments, the humectant is selected from sorbitol, glycerin, xylitol, polyethylene glycol, propylene glycol, and combinations thereof. In some embodiments, the humectant is sorbitol. In some embodiments, the humectant is glycerin. In some embodiments, the humectant is present in an amount of from 5 to 25 weight %; from 7 to 23 weight %; from 10 to 15 weight %; about 15 weight %; or about 13 weight %, based on the total weight of the composition. When the humectant is supplied as a solution in water, for example sorbitol as a 70 weight % solution in water, the amount of humectant is calculated as the active weight of the humectant, e.g. for a composition comprising 25 weight % sorbitol (as 70 weight% aqueous solution), the concentration of humectant is 17.5 weight %.

In some embodiments, the compositions have a pH of from 8.5 to 10.5; or from 9.2 to 10.2. In certain embodiments, the compositions further comprise a buffer system, which may be: (a) a combination of sodium silicate and tetrasodium pyrophosphate; (b) a combination of sodium hydroxide, sodium bicarbonate and tetrasodium pyrophosphate; or (c) a combination of sodium bicarbonate and sodium carbonate. In some embodiments, the buffer system is 0.25 to 0.75 weight % sodium silicate and 0.25 to 0.75 weight % tetrasodium pyrophosphate; or about 0.4 weight % sodium silicate and about 0.5 weight % tetrasodium pyrophosphate, based on the total weight of the composition. Various grades of sodium silicate are characterized by their $SiO_2:Na_2O$ ratio, which can vary between 1:2 and 1:3.75 by weight. Grades with this ratio being greater than 1:2.85 by weight are termed "alkaline". An example of a sodium silicate useful in the present invention is sodium silicate with target pH of 8.5, which has a $SiO_2:Na_2O$ ratio of 1:3.26 by weight and a relative density of 41 BE (BE denoting "Baume") and is denoted as "sodium silicate 1:3.26-41BE".

In some embodiments, the buffer system is 0.03 to 0.5 weight % sodium hydroxide, 0.25 to 0.75 weight % sodium bicarbonate and 0.25 to 1.5 weight % tetrasodium pyrophosphate; or about 0.04 weight % sodium hydroxide, about 0.5 weight % sodium bicarbonate and about 0.5 weight % tetrasodium pyrophosphate, based on the total weight of the composition. Optionally, the buffer system is 0.05 to 0.5 weight % sodium bicarbonate and 0.2 to 0.6 weight % sodium carbonate; or 0.1 weight % sodium bicarbonate and 0.4 weight % sodium carbonate, based on the total weight of the composition.

In some embodiments, the composition is a toothpaste, a tooth gel, or a combination thereof.

In some embodiments, the viscosity of the oral care composition is from 100,000 to 1,000,000; from 190,000 to 800,000 cps; from 200,000 to 800,000 cps; from 200,000 to 700,000 cps; from 300,000 to 600,000 cps; or from 300,000 to 500,000 cps, as measured at 25°C at 1 rpm using a Brookfield Viscometer Model HADV-II + Pro and a V74 spindle. In some embodiments, the viscosity of the oral care composition immediately following its formation is from 100,000 to 1,000,000; from 190,000 to 800,000 cps; from 200,000 to 800,000 cps; from 200,000 to 700,000 cps; from 300,000 to 600,000 cps; or from 300,000 to 500,000 cps, as measured at 25°C at 1 rpm using a Brookfield Viscometer Model HADV-11 + Pro and a V74 spindle. In some embodiments, the viscosity of the oral care composition after storing for 1 day (24 hours) at 25°C/60% relative humidity (RH) in a sealed 5 fl. oz. laminate tube is from 100,000 to 1,000,000; from 190,000 to 800,000 cps; from 200,000 to 800,000 cps; from 200,000 to 700,000 cps; from 300,000 to 600,000 cps; or from 300,000 to 500,000 cps, as measured at 25°C at 1 rpm using a Brookfield Viscometer Model HADV-II + Pro and a V74 spindle. In some embodiments, the viscosity of the oral care composition after storing for 3 days at 25°C/60% relative humidity (RH) in a sealed 5 fl. oz. laminate tube is from 100,000 to 1,000,000; from 190,000 to 800,000 cps; from 200,000 to 800,000 cps; from 200,000 to 700,000 cps; from 300,000 to 600,000 cps; or from 300,000 to 500,000 cps, as measured at 25°C at 1 rpm using a Brookfield Viscometer Model HADV-II + Pro and a V74 spindle. In some embodiments, the viscosity of the oral care composition after storing for 1 week at 25°C/60% relative humidity (RH) in a sealed 5 fl. oz. laminate tube is from 100,000 to 1,000,000; from 190,000 to 800,000 cps; from 200,000 to 800,000 cps; from 200,000 to 700,000 cps; or from 300,000 to 600,000 cps, as measured at 25°C at 1 rpm using a Brookfield Viscometer Model HADV-11 + Pro and a V74 spindle. In some embodiments, the viscosity of the oral care composition after storing for 1 month at 25°C/60% relative humidity (RH) in a sealed 5 fl. oz. laminate tube is from 100,000 to 1,000,000; from 190,000 to 800,000 cps; from 200,000 to 800,000 cps; from 200,000 to 700,000 cps; from 300,000 to 600,000 cps; or from 400,000 to 600,000 cps, as measured at 25°C at 1 rpm using a Brookfield Viscometer Model HADV-II + Pro and a V74 spindle. In some embodiments, the viscosity of the oral care composition after storing for 2 months at 25°C/60% relative humidity (RH) in a sealed 5 fl. oz. laminate tube is from 100,000 to 1,000,000; from 190,000 to 800,000 cps; from 200,000 to 800,000 cps; from 200,000 to 700,000 cps; from 300,000 to 600,000 cps; or from 400,000 to 600,000 cps, as measured at 25°C at 1 rpm using a Brookfield Viscometer Model HADV-II + Pro and a V74 spindle. In some embodiments, the viscosity of the oral care composition after storing for 3 months at 25°C/60% relative humidity (RH) in a sealed 5 fl. oz. laminate tube is from 100,000 to 1,000,000; from 190,000 to 800,000 cps; from 200,000 to 800,000 cps; from 200,000 to 700,000 cps; from 400,000 to 500,000 cps, as measured at 25°C at 1 rpm using a Brookfield Viscometer Model HADV-II + Pro and a V74 spindle. The methodology for measuring the viscosity is discussed in Example 3, below.

In some embodiments, the composition has a static yield stress (YS) of at least 50 Pa; from 50 to 320 Pa; or from 80 to 290 Pa, as measured at 25°C using a Brookfield Viscometer Model HADV-II + Pro and a V74 spindle. In some embodiments, the static yield stress of the composition immediately following its formation is at least 50 Pa; from 50 to 320 Pa; from 50 to 150 Pa; or from 80 to 140 Pa, as measured at 25°C using a Brookfield Viscometer Model HADV-II + Pro and a V74 spindle. In some embodiments, the composition has a static yield stress of at least 50 Pa; from 50 to 320 Pa; from 50 to 200 Pa; from 50 to 150 Pa as measured at 25°C using a V74 spindle after storing for 1 day (24 hours) at 25°C/60% relative humidity (RH) in a sealed 5 fl. oz. laminate tube. In some embodiments, the composition has a static yield stress of at least 50 Pa; from 50 to 320 Pa; from 70 to 320 Pa; or from 100 to 200 Pa as measured at 25°C using a Brookfield Viscometer Model HADV-II + Pro and a V74 spindle after storing for 3 days at 25°C/60% relative humidity (RH) in a sealed 5 fl. oz. laminate tube. In some embodiments, the composition has a static yield stress of at least 50 Pa; from 80 to 320 Pa; or from 120 to 290 Pa, as measured at 25°C using a Brookfield Viscometer Model HADV-11 + Pro and a V74 spindle after storing for 1 week at 25°C/60%) relative humidity (RH) in a sealed 5 fl. oz. laminate tube. In some embodiments, the composition has a static yield stress of at least 50 Pa; from 90 to 310 Pa; or from 200 to 300 Pa, as measured at 25°C using a Brookfield Viscometer Model HADV-11 + Pro and a V74 spindle after storing for 1 month at 25°C/60% relative humidity (RH) in a sealed 5 fl. oz. laminate tube. In some embodiments, the composition has a static yield stress of at least 50 Pa; from 100 to 300 Pa; or from 200 to 290 Pa, as measured at 25°C using a Brookfield Viscometer Model HADV-II + Pro and a V74 spindle after storing for 2 months at 25°C/60% relative humidity (RH) in a sealed 5 fl. oz. laminate tube. In some embodiments, the composition has a static yield stress of at least 50 Pa; from 100 to 320 Pa; from 200 to 300 Pa; or about 290 Pa, as measured at 25°C using a Brookfield Viscometer Model HADV-II + Pro and a V74 spindle after storing for 3 months at 25"C/60% relative humidity (RH) in a sealed 5 fl. oz. laminate tube. The methodology for measuring static yield stress is discussed in Example 3, below.

In a third aspect, the present invention provides for use, in an oral care composition, of a combination of talc and calcium carbonate to improve flavor release.

In some embodiments, the calcium carbonate is present in the oral care composition in an amount of from 15 to 35 weight %, and the talc is present in the oral care composition in an amount of from 5 to 25 weight %, based on the total weight of the composition. In some embodiments, the calcium carbonate is present in an amount of 16 to 34 weight %; from 17 to 33 weight %; from 18 to 32 weight %; from 19 to 31 weight %; from 20 to 30 weight %; about 20 weight %; or about 30 weight %, based on the total weight of the composition. In some embodiments, the talc is present in an amount of from 6 to 24 weight %; from 7 to 23 weight %; from 8 to 22 weight %; from 9 to 21 weight %; from 10 to 20 weight %, about 10 weight %; about 20 weight %; from 12 to 18 weight %; from 14 to 16 weight %; or about 15 weight %, based on the total weight of the composition. In certain embodiments, the total concentration of talc and calcium carbonate in the oral care composition is greater than 35 weight %; from greater than 35 weight % to 55 weight %, from greater than 35 weight % to 50 weight %, from greater than 35 weight % to 45 weight %, from greater than 35 weight % to 42 weight %, or from greater than 35 weight % to 40 weight %, by total weight of the composition.

In certain embodiments, the calcium carbonate is present in the oral care composition in an amount of from 15 to 30 weight %, and the talc is present in an amount of from 5 to 20 weight %, based on the total weight of the composition. In some embodiments, the oral care composition comprises from 16 to 25 weight %; from 17 to 23 weight %; from 18 to 22 weight %; from 19 to 21 weight %; or about 20 weight % calcium carbonate, based on the total weight of the composition. In some embodiments, the talc is present in an amount of 6 to 18 weight %; from 7 to 15 weight %; from 8 to 12 weight %; from 9 to 10 weight %; or about 10 weight %, based on the total weight of the composition. In some embodiments, the total concentration of talc and calcium carbonate in the oral care composition is less than 35 weight %, based on the total weight of the composition, and wherein the composition comprises an additional structuring agent which comprises at least one of: (i) a cellulose ether thickening agent; and (ii) an inorganic thickening agent selected from thickening silica, a clay thickening agent, and mixtures thereof. In some embodiments, the oral care composition comprises from 0.5 to 2 weight %; from 1.6 to 2 weight %; from 1.6 to 1.8 weight %; 1.6 weight %; or 1.8 weight % cellulose ether thickening agent, based on the total weight of the composition. In certain embodiments, the oral care composition comprises from 1 to 6 weight %; from 2 to 5 weight %; from 4 to 5 weight %; about 5 weight %; about 4 weight %; or about 2 weight % thickening silica, based on the total weight of the composition. In some embodiments, the oral care composition comprises from 0.5 to 2 weight %; from 1 to 2 weight %; or about 1 weight % clay thickening agent, based on the total weight of the composition.

In any of the above embodiments of the third aspect of the present invention, the calcium carbonate, talc, cellulose ether thickening agent, thickening silica and clay thickening agent may be any of those as described above for the compositions of the first and second aspects.

The present invention also provides for use, in an oral care composition, of a combination of talc and calcium carbonate to improve flavor release, wherein the oral care composition is a composition as described above in any of the above embodiments of the first and second aspects of the present invention.

The oral care compositions of the present invention may further comprise additional ingredients. These additional ingredients may include, but are not limited to, diluents, bicarbonate salts, surfactants, foam modulators, sweeteners, flavorants, pigments, antibacterial agents, anticaries agents, anticalculus or tartar control agents, and mixtures thereof.

In some embodiments, the oral care compositions of the present invention comprise at least one bicarbonate salt useful for example to impart a "clean feel" to teeth and gums due to effervescence and release of carbon dioxide. Any orally acceptable bicarbonate can be used, including without limitation, alkali metal bicarbonates such as sodium and potassium bicarbonates, ammonium bicarbonate and the like. The one or more additional bicarbonate salts are optionally present in a total amount of about 0.1 wt. % to about 50 wt. %, for example about 1 wt. % to 20 wt. %, by total weight of the composition.

The oral care compositions of the invention may also comprise at least one surfactant. Any orally acceptable surfactant, most of which are anionic, nonionic or amphoteric, can be used. Suitable anionic surfactants include without limitation, water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium coconut monoglyceride sulfonate, sodium lauryl sarcosinate, sodium lauryl isethionate, sodium laureth carboxylate and sodium dodecyl benzenesulfonate. Suitable nonionic surfactants include without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation, derivatives of $C_{8-20}$ aliphatic secondary and tertiary amines having an anionic group such as carboxylate, sulfate, sultanate, phosphate or phosphonate. Betaines may also be used, a suitable example of which is cocoamidopropyl betaine. One or more surfactants are optionally present in a total amount of about 0.01 wt.% to about 10 wt. %, for example, from about 0.05 wt. % to about 5 wt. %, or from about 0.1 wt. % to about 2 wt. % by total weight of the composition.

The oral care compositions of the invention may comprise at least one foam modulator, useful for example to increase amount, thickness or stability of foam generated by the composition upon agitation. Any orally acceptable foam modulator can be used, including without limitation, polyethylene glycols (PEGs), also known as polyoxyethylenes. High molecular weight PEGs are suitable, including those having an average molecular weight of 200,000 to 7,000,000, for example 500,000 to 5,000,000, or 1,000,000 to 2,500,000. One or more PEGs are optionally present in a total amount of about 0.1 wt. % to about 10 wt. %, for example from about 0.2 wt. % to about 5 wt. %, or from about 0.25 wt. % to about 2 wt.%, by total weight of the composition.

The oral care compositions of the present invention may comprise at least one sweetener (such as, for example, sodium saccharin), useful for example to enhance taste of the composition. One or more sweeteners are optionally present in a total amount depending strongly on the particular sweetener(s) selected, but typically 0.005 wt.% to 5 wt.%, by total weight of the composition, optionally 0.005 wt.% to 0.2 wt.%, further optionally 0.05 wt.% to 0.1 wt.% by total weight of the composition.

The compositions of the present invention may also comprise at least one flavorant, useful for example to enhance taste of the composition. Any orally acceptable natural or synthetic flavorant can be used, including without limitation tea flavours, vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, *etc.*, bean- and nut-derived flavors such as coffee, cocoa, cola, peanut, almond, *etc.*, adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, α-irisone, propenyl guaiethol, thymol, linalool, benzaldehyde, cinnamaldehyde, N-ethyl-*p*-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from about 0.01 wt. % to about 5 wt. %, for example, from about 0.03 wt. % to about 2.5 wt.%, optionally about 0.05 wt.% to about 1.5 wt.%, further optionally about 0.1 wt.% to about 0.3 wt.% by total weight of the composition.

The compositions of the invention may comprise at least one colorant. Colorants herein include pigments, dyes, lakes and agents imparting a particular luster or reflectivity such as pearling agents. Any orally acceptable colorant can be used, including without limitation titanium dioxide, zinc oxide, red, yellow, brown and black iron oxides, ferric ammonium ferrocyanide, manganese violet, ultramarine, titaniated mica, bismuth oxychloride, and the like. One or more colorants are optionally present in a total amount of from about 0.001 wt.% to about 20 wt.%, for example, from about 0.01 wt.% to about 10 wt. %, or from about 0.1 wt. % to about 5 wt.%, by total weight of the composition.

The oral care compositions may also comprise a fluoride ion source. Fluoride ion sources include, but are not limited to: stannous fluoride, sodium fluoride, potassium fluoride, potassium monofluorophosphate, sodium monofluorophosphate (NaMFP), ammonium monofluorophosphate, sodium fluorosilicate, ammonium fluorosilicate, amine fluoride such as olaflur (N'-octadecyltrimethylendiamine-N,N,N'-tris(2-ethanol)"dihydrofluoride), ammonium fluoride, and combinations thereof. In certain embodiments the fluoride ion source includes stannous fluoride, sodium fluoride, amine fluorides, sodium monofluorophosphate, as well as mixtures thereof In certain embodiments, the oral care composition of the invention may also contain a source of fluoride ions or fluorine-providing ingredient in amounts sufficient to supply about 50 to about 5000 ppm fluoride ion, e.g., from about 100 to about 1000, from about 200 to about 500, or about 250 ppm fluoride ion. Fluoride ion sources may be added to the compositions of the invention at a level of about 0.001 wt. % to about 10 wt. %, e.g., from about 0.003 wt. % to about 5 wt. %, 0.01 wt. % to about 1 wt., or about 0.05 wt. %. However, it is to be understood that the weights of fluoride salts to provide the appropriate level of fluoride ion will obviously vary based on the weight of the counter ion in the salt, and one of skill in the art may readily determine such amounts. A preferred fluoride salt may be sodium fluoride.

The compositions of the present invention may comprise a saliva stimulating agent useful, for example, in amelioration of dry mouth. Any orally acceptable saliva stimulating agent can be used, including without limitation food acids such as citric, lactic, malic, succinic, ascorbic, adipic, fumaric and tartaric acids, and mixtures thereof. One or more saliva stimulating agents are optionally present in saliva stimulating effective total amount.

The compositions of the present invention may include antisensitivity agents, e.g., potassium salts such as potassium nitrate, potassium bicarbonate, potassium chloride, potassium citrate, and potassium oxalate; capsaicin; eugenol; strontium salts; chloride salts and combinations thereof. Such agents may be added in effective amounts, e.g., from about 1 wt. % to about 20 wt. % by weight based on the total weight of the composition, depending on the agent chosen.

The composition of the invention may further comprise an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

The compositions of the present invention may additionally optionally comprise a tartar control (anticalculus) agent as provided bellow. Tartar control agents among those useful herein include salts of the specified agents, including alkali metal and ammonium salts. The agents include: phosphates and polyphosphates, polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, sodium trimetaphosphate, sodium hexametaphosphate and mixtures thereof. Other useful tartar control agents include polycarboxylate polymers and polyvinyl methyl ether/maleic anhydride (PVM/MA) copolymers, such as GANTREZ®.

**EXAMPLES**

Example 1

Natural talc of USP grade from several different supply sources was compared for physical and chemical characteristics. X-ray diffraction (PXRD) analysis confirms the absence of asbestos or crystalline $SiO_2$ phases with all samples. Also, PXRD measurements revealed that composition varies somewhat for the different natural supply sources, with chlorite, quartz, calcite and dolomite being the typical "impurity" materials. These impurities can be of different proportions and include other minerals depending on mine location and methods of separation and purification.

Scanning electron microscopy (SEM) evaluations were conducted to study the morphology of talc powder from various natural supplies. Observations, and subsequent conclusions, were made on each individual sample after examining various locations and recording images of those regions which were representative in nature of that sample. The samples were defined to have one of the following morphologies: plate-like and smooth (highly lamellar); not plate-like and rough (compact); or a combination of the two. The size of an individual talc platelet, which is a few thousand elementary sheets, can vary from approximately one micron to over one hundred microns depending on the deposit. It is this individual platelet size that determines talc's lamellarity. Highly lamellar talc (macrocrystalline talc) has large individual platelets, whereas compact talc (microcrystalline talc) has much smaller platelets.

Toothpaste prototypes were formulated with each of these materials and all finished products were found to be acceptable for chemical, physical and organoleptic aspects. However, natural talc of macrocrystalline morphology and higher purity, when compared to equivalent dosages of other talc of microcrystalline morphology and lower purity, provided the greatest viscosity and static yield stress in toothpaste formulations, (containing calcium carbonate), and was thus found to provide the best ribbon and stripe quality and the best overall product physical stability. Without being bound by any theory, it is believed that this is likely to be due to a combination of talc chemical composition, particle size and particle morphology, and resulting packing interaction with calcium carbonate abrasive.

Example 2

Formulations containing different concentrations of calcium carbonate abrasive were compared for mechanical cleaning potential by means of *in vitro* pellicle cleaning ratio (PCR) scores. A critical calcium carbonate loading was identified for delivering mechanical cleaning of teeth which is comparable to benchmark marketed formulas. Prototypes formulated with at least 20% precipitated or natural calcium carbonate provide statistically equivalent cleaning potential to respective benchmark formulations containing 40% PCC and 42% NCC, respectively (see Tables 1 and 2, below). Also, Talc was found in *in vitro* studies to have no impact (does not provide a cleaning benefit) on PCR scores. Without being bound by any theory, it is believed that this may arise due to the low hardness rating of talc on the Mohs hardness scale (hardness rating of 1). While it does not provide a cleaning contribution, talc is less costly than calcium carbonate and so replacing some of the calcium carbonate with talc may provide a cost savings benefit.

**Table 1**

| Formula | i | ii | iii | Benchmark |
|---|---|---|---|---|
| Ppt. Calcium Carbonate - PCC | 10.00 | 20.00 | 30.00 | 40.00 |
| **PCR Score\*** | 72[b] | 80[a] | 80[a] | 80[a] |
| **\*(PCR scores with the same superscript letter are not significantly different as per one-way ANOVA (Analysis of Variance) and SNK (Student-Newman-Keuls) analyses = P>0.05)** | | | | |

**Table 2**

| Formula | iv | v | vi | Benchmark |
|---|---|---|---|---|
| Nat. Calcium Carbonate - NCC | 10.00 | 20.00 | 30.00 | 42.00 |

(continued)

| Formula | iv | v | vi | Benchmark |
|---|---|---|---|---|
| PCR Score* | 85[b] | 95[a] | 97[a] | 105[a] |

*(PCR scores with the same superscript letter are not significantly different, as per one-way ANOVA (Analysis of Variance) and SNK (Student-Newman-Keuls) analyses = P>0.05)

An in vitro model has been developed for evaluating the ability of dentifrices to clean extrinsic (surface) stains. Through use of a stained film deposited on enamel sections, a comparison of test formulations to a standard ADA (American Dental Association) abrasion reference material (slurry of calcium pyrophosphate) can be established. The ratio of the test formulation to the standard is the pellicle cleaning ratio (PCR). (Reference: Stookey, G. K. et al, "In vitro removal of stain with dentifrice", Journal of Dental Research, Vol. 61, no. 11, November 1982, p. 1236-1239). The results in tables 1 and 2, above, were obtained using this method.

Example 3

Certain characteristics of toothpastes are important for consumer acceptance. These include, for example, ease of product dispensing (from a tube) and standup/ribbon quality. These attributes are greatly influenced by the viscosity and static yield stress of the compositions. It is desirable to closely match the viscosity and static yield stress (YS) profiles of the test compositions with reduced calcium carbonate levels to those of the "benchmark" products, so as to provide the compositions with similar characteristics to those of the "Benchmark" products, and thus to prevent there being a noticeable difference to consumers in these characteristics. Furthermore, targeted viscosity and yield stress characteristics ensure acceptable product stability and bulk product transfer behavior during manufacturing. Cost savings may also be achieved by using talc, as a result of the reduction of the level of humectants required in the talc-containing formulas.

The effect of varying the concentration of precipitated calcium carbonate (PCC) and talc (magnesium silicate) in a dentifrice on the viscosity, static yield stress and overall physical stability thereof (as compared to a "benchmark" calcium carbonate-based toothpaste which contained 40 weight % PCC, 0.80 weight % NaCMC and no talc) was studied.

The formulae tested are shown in Table 3, below:

**Table 3**

| Formula # | A | B | C | D | E (Benchmark) |
|---|---|---|---|---|---|
| Ppt. Calcium Carb. - PCC | 30.00 | 20.00 | 20.00 | 20.00 | 40.00 |
| Magnesium silicate (Talc) | 10.00 | 20.00 | 15.00 | 10.00 | 0.00 |
| NaCMC Type 7 | 1.00 | 1.25 | 1.60 | 1.80 | 0.80 |
| Thickener Silica | 0 | 0 | 2.00 | 5.00 | 0 |
| Glycrin, Vegetable | 13.00 | 13.00 | 13.00 | 13.00 | 12.00-18.00 |
| Tetrasodium Pyrophosphate | 0.50 | 0.50 | 0.50 | 0.50 | 0.25-0.75 |
| Sodium Bicarbonate | 0.50 | 0.50 | 0.50 | 0.50 | 0.25-0.75 |
| Sodium Hydroxide (38 wt.% aq Sol'n) | 0.10 | 0.10 | 0.10 | 0.10 | 0.03-0.50 |
| Demineralized Water | 40.64 | 40.69 | 43.34 | 45.14 | 35.00-47.00 |
| NaMFP - USP | 1.10 | 1.10 | 1.10 | 1.10 | 1.00-1.50 |
| Sodrium Lauryl Sulfate | 1.76 | 1.76 | 1.76 | 1.76 | 1.00-2.00 |
| Sodium Saccharin | 0.25 | 0.25 | 0.25 | 0.25 | 0.10-1.00 |
| Flavor | 0.85 | 0.85 | 0.85 | 0.85 | 0.95 |
| Benzyl Alcohol | 0.30 | 0.30 | 0.30 | 0.30 | 0.10-1.00 |
| Viscosity (x10,000 cps)/YS (Pa) @ RT, V74 spindle | | | | | |
| Initial | 36 / 155 | 33 / 129 | 30 / 28 | 53 / 16 | 24 / 128 |
| 1 day | 46 / 290 | 33 / 129 | 36 / 46 | 43 / 38 | 41 / 290 |
| 3 days | 41 / 290 | 38 / 193 | 44 / 58 | 50 / 52 | 44 / 290 |
| 1 Week | 53 / 290 | 35 / 2.55 | 48 / 85 | 52 / 58 | 47 / 319 |
| 1 Month | 54 / 290 | 46 / 255 | 48 / 85 | 55 / 42 | 56 / 353 |
| 2 Months | 56 / 290 | 46 / 290 | 56 / 81 | 55 / 46 | 58 / 353 |

(continued)

| Viscosity (x10,000 cps)/YS (Pa) @ RT, V74 spindle | | | | | |
|---|---|---|---|---|---|
| 3 Months | 60 / 290 | 46 / 290 | 50 / 76 | 55 / 52 | 58 / 353 |
| Separation Score | | | | | |
| | 2.40 (Pass) | 2.48 (Pass) | 2.89 (Pass) | 2.95(Pass) | 2.48 (Pass) |
| pH (10% in water) | | | | | |
| Initial | 9.58 | 9.71 | 9.61 | 9.57 | 9.61 |
| 3 months@25°C/60%RH | 9.59 | 9.71 | 9.56 | 9.54 | 9.61 |
| 3 months@40°C/75%RH | 9.44 | 9.65 | 9.43 | 9.42 | 9.60 |
| Soluble F$^-$, ppm | | | | | |
| Initial | 1445 | 1440 | 1440 | 1500 | 1455 |
| 3months@25°C/60%RH | 1218 | 1370 | 1312 | 1390 | 1038 |
| 3 months@40°C/75%RH | 977 | 1050 | 985 | 1010 | 913 |
| Specific Gravity | | | | | |
| | 1.46 | 1.44 | 1.43 | 1.41 | 1.45 |

The viscosity of the above compositions was measured immediately after their formation ("initial"), and again after 1 day, 3 days, 1 week, 1 month, 2 months and 3 months of storage. The storage conditions were 25°C and 60 % relative humidity (RH), and the compositions were stored in sealed 5 fl. oz. laminate tubes with the tubes filled to capacity. The viscosity measurements were made using a Brookfield Viscometer model HADV-II + pro and a V74 spindle, at 1 rpm and at 25°C.

The static yield stress (YS) of the above compositions was also measured immediately after their formation ("initial"), and again after 1 day, 3 days, 1 week, 1 month, 2 months and 3 months of storage at 25°C and 60 % relative humidity, with the compositions being stored in sealed 5 fl. oz. laminate tubes with the tubes filled to capacity. The static yield stress measurements were made using a Brookfield Viscometer model HADV-II + Pro and a V74 spindle, at 25°C.

The viscosity and static yield stress were measured on a Brookfield HADVII+Pro viscometer with spindle V-74 available from Brookfield Engineering Laboratories. All measurements on this viscometer were performed at room temperature (25°C). In the tests, the spindle is rotated at a pre-set RPM (rotations per minute) series, while torque (Torque%) is reported in terms of % of its maximum (Tmax) as specified for the instrument (Tmax = 1.437 mN·m for HADVII+Pro). Only those measurements with Torque% between 10 and 100% of Tmax are valid. The raw data (Torque% and RPM) were converted into shear stress (SS) and shear rate (SR) using well known formulas for Couette geometry, assuming that the vane (spindle) performs as its encompassing cylinder (i.e., assuming that paste between its blades move as a solid piece):

$$SR = SRC*RPM$$

$$SS = SF*Torque\%$$

where

$$SRC = (\pi/15)C/(1-x^2)$$

$$SF = 0.01*Tmax*C/(2\,\pi LR^2)$$

$$C = (1+x^2)/2,$$

where L is the vane blade length and R is the vane radius; and x is the ratio of spindle diameter to the diameter of the vessel in which the measurement is performed. (* denotes multiplication). Here L and R are in meters, Tmax is in N·m, SS is in Pascals and SR is in reciprocal seconds.

In the test, RPM was swept from 0.5 to 200 in 20 steps in logarithmical mode, 10 sec per step. "viscosity" reported herein refers to SS/SR at 1 RPM (reported herein in centipoise, i.e. cps, wherein 1 cps = 0.001Pa·s). Furthermore, SS(SR) function was fitted with Casson equation, $SS = (Y^n+(V0*SR)^n)^{1/n}$ where Y, V0 and n are fitting parameters. Only the monotonously increasing section of the curve bended upward (i.e., the one in which both first and second derivative of SS(SR) were positive, typically above 1 RPM) was fitted. "Yield stress" reported herein refers to Y parameter.

The physical stability of each formula was also evaluated, i.e. the degree of separation of the mixture into two or more phases/layers after initial formation and after storage in sealed tubes for 3 months at three different temperature conditions (25°C/60% relative humidity; 40°C/75% relative humidity; and 49°C). The physical stability of the formulas after 3 months aging at 40°C and 75% relative humidity is reported in Table 3, above ("Separation Score"). This high-temperature aging is used as a predictive measure, and has been found to correlate well with two years' shelf-life determination under 25°C/60% relative humidity conditions. In these evaluations, a trained evaluator visually examines several attributes of the compositions under test, and provides a numeric rating of 0 to 4 (0 = no separation; 1 = slight separation; 2 = minor; 3 = moderate; 4 = severe) for each attribute for each time point and for each set of storage conditions. The attributes are measured for: (1) a ribbon of the toothpaste squeezed from the tube (ribbon stand-up, cap separation, aeration, lumps/grit, graininess, discoloration); (2) tube cut open (appearance, aeration, separation, wall separation, clip separation, pocket, discoloration). The composition passes this visual inspection test if it achieved a rating of 3 or less in all of the indicated attributes. The average score specifically related to wall separation across at least three tubes for each formulation is reported in Table 3, above (as the "Separation Score"), with a score of three or less being acceptable ("pass") and a score of greater than three being unacceptable ("fail").

The concentration of soluble fluorite ion (in ppm) present in the compositions, and the pH of a 10 weight % solution of the compositions in deionized water, were measured immediately after the formation of the compositions, and again after one, two and three months aging at controlled room temperature (25°C and 60% relative humidity) and accelerated high temperature conditions (40°C and 75 % relative humidity) in sealed 5 fl. oz. laminate tubes with the tubes filled to capacity. The results for the initial measurement and the measurements at three months are reported in Table 3, above.

The specific gravity of the formulations was also measured immediately after the formation of the compositions. A gravimetric method was utilized where a cylinder of known mass and volume was filled to capacity with the test product. The cylinder filled with test product was then weighed and the mass of the cylinder was then subtracted from the total mass to obtain the mass of the test product. The mass of the test product (in grams) was then divided by the volume of the cylinder (in milliliters) to obtain the specific gravity of the test product. The measurement was done at 25°C and atmospheric pressure. The specific gravity of the formulations is reported in Tables 3 and 4 as the specific gravity in relation to water, which has a specific gravity of 1 when measuring mass in grams and volume in milliliters (cubic centimeters) at 25°C and atmospheric pressure.

Formulas containing either sorbitol or glycerin as the humectant, and either natural calcium carbonate (NCC) or precipitated calcium carbonate (PCC) as the abrasive were also developed. The pH of these formulas was targeted to between 9.2 and 10.2 in order to ensure chemical stability of the calcium carbonate-containing formulas. Different buffering systems were utilized to maintain this pH range. The combinations of: 1) sodium silicate and tetrasodium pyrophosphate; or 2) sodium hydroxide, sodium bicarbonate and tetrasodium pyrophosphate; or 3) sodium carbonate and sodium bicarbonate were found to be effective buffer systems for these formulas. Also, addition of benzyl alcohol was found to effectively enhance robustness to potential microbial contamination in these high water formulations.

The viscosity, yield stress and Separation Score of these compositions were measured using the same methods/protocols as described above.

The concentration of soluble fluoride ion (in ppm) present in the compositions, and the pH of a 10 weight % solution of the compositions in deionized water, were measured immediately after the formation of the compositions, and again after one, two and three months aging at controlled room temperature (25°C and 60% relative humidity) and accelerated high temperature conditions (40°C and 75 % relative humidity) in sealed 5 fl. oz. tubes, in order to evaluate the chemical stability of the formulations. The results for the initial measurement and the measurement at three months are reported below.

The specific gravity of the compositions was also measured, as detailed above.

The formulas tested and their results are shown in Table 4, below.

**Table 4**

| Formula # | F | G | H | I | J (Benchmark) |
|---|---|---|---|---|---|
| Nat. Calcium Carb. NCC | 30.00 | 20.00 | 20.00 | 20.00 | 42.00 |
| Magnesium silicate (Talc) | 10.00 | 20.00 | 15.00 | 10.00 | 0.00 |

(continued)

| Formula # | F | G | H | I | J (Benchmark) |
|---|---|---|---|---|---|
| NaCMC Type 8 | 1.30 | 1.50 | 1.60 | 1.60 | 1.00 |
| Thickener Silica | 0 | 0 | 2.00 | 4.00 | 2.00 |
| Magnesium Aluminum Silicate | 0 | 0 | 1.00 | 1.00 | 1.00 |
| Sorbitol, (Non Crystallizing). (70 wt.% aq soln | 21.00 | 21.00 | 21.00 | 21.00 | 15.00-25.00 |
| Sodium Carbonate (Soda Ash) | 0.40 | 0.40 | 0.40 | 0.40 | 0.20-0.60 |
| Sodium Bicarbonate | 0.10 | 0.10 | 0.10 | 0.10 | 0.05-1.00 |
| Demineralized Water | 32.95 | 32.75 | 34.65 | 37.65 | 25.00-35.00 |
| NaMFP-USP | 1.10 | 1.10 | 1.10 | 1.10 | 1.00-1.50 |
| Sodium Lauryl Sulfate | 1.90 | 1.90 | 1.90 | 1.90 | 1.00-2.00 |
| Sodium Saccharin | 0.15 | 0.15 | 0.15 | 0.15 | 0.1-1.00 |
| Flavor | 0.80 | 0.80 | 0.80 | 0.80 | 1.00 |
| Benzyl Alcohol | 0.30 | 0.30 | 0.30 | 0.30 | 0.10-1.00 |
| Viscosity (x10,000 cps)/YS (Pa) @ RT, V74 spindle | | | | | |
| Initial | 40 / 95 | 42 / 57 | 46/46 | 48/39 | 51/290 |
| 1 day | 44 / 190 | 46 / 76 | 50/96 | 50/39 | 49/290 |
| 3 days | 49 / 190 | 51 / 76 | 53/113 | 40/39 | 53/290 |
| 1 Week | 55 / 233 | 56 / 12.8 | 52/142 | 42/86 | 53/319 |
| 1 Month | 62 / 290 | 56 / 233 | 56/170 | 41/104 | 55/319 |
| 2 Months | 58 / 290 | 56 / 233 | 55/170 | 41/117 | 57/319 |
| 3 months | 62 / 290 | 56 / 233 | 55/170 | 41/142 | 59/319 |
| Separation Score | | | | | |
| | 2.28 (Pass) | 2.57 (Pass) | 2.48 (Pass) | 2.76 (Pass) | 2.16 (Pass) |
| pH (10% in water) | | | | | |
| Initial | 9.80 | 10.30 | 9.80 | 9.90 | 10.00 |
| 3 months@25°C/60%RH | 9.71 | 10.24 | 9.73 | 9.77 | 9.80 |
| 3 months@40°C/75%RH | 9.46 | 10.05 | 9.61 | 9.45 | 9.58 |
| Soluble F$^-$, ppm | | | | | |
| Initial | 1445 | 1440 | 1440 | 1450 | 1480 |
| 3 months@25°C/60%RH | 1404 | 1400 | 1412 | 1400 | 1430 |
| 3 months@40°C/75%RH | 1264 | 1390 | 1275 | 1250 | 1350 |
| Specific Gravity | | | | | |
| | 1.50 | 1.46 | 1.44 | 1.43 | 1.52 |

It can be seen from Tables 3 and 4 that all of the formulas pass the visual evaluation test (Separation Score). However, those formulations with a lower total concentration of talc and calcium carbonate (35 weight % or less) show signs of being less robust in terms of physical stability than those formulations with a higher total concentration of talc and calcium carbonate.

It was found that, for desirable physical attributes described above, a critical solids level (i.e. total concentration of calcium carbonate and talc) of greater than 35 weight % to approximately 42 weight % should preferably be maintained when replacing calcium carbonate with talc as shown in Table 4, formulas A and B, and in Table 5, formulas F and G. Formulas with 35 weight % or less total talc/calcium carbonate solids are possible but only with significant addition of other structuring agents such as gum and/or thickener silica as shown in Table 3, formulas C and D, and in Table 4, formulas H and I. However, these formulas with reduced solids and increased thickeners display rheology profiles less similar to the benchmarks particularly in terms of static yield stress. These formulas may also be less cost-effective than compositions with higher talc/calcium carbonate loading and lower concentrations of thickeners.

Example 4

In addition to cost savings benefits, Talc/PCC and Talc/NCC formulations of the present invention show a reduced propensity to dry out with prolonged exposure to air, as compared to calcium carbonate-only benchmarks. This is an important advantage as calcium carbonate formulations in general tend to exhibit rapid dryout if consumers are not vigilant in closing the cap of the toothpaste tube after usage. This may result in consumer dissatisfaction related to product becoming more difficult to dispense from increased viscosity and tube orifice plugging, loss of flavor and increased mess with cracked/ crumbling product.

A rheology test has been developed to quantify toothpaste dry-out when exposed to air. Dry-out is represented by an increase in relative elastic modulus over time as measured by a surface probe. More specifically, the surface probe is a de Nouy ring (RI 01 by Kruss) and it is mounted on a TA ARG2 rheometer with an attachment (supplied by TA Instruments) for surface rheology. In this evaluation, a test sample was deposited on the rheometer Peltier plate at 30°C using a plastic bounding ring and leveled with a spatula so as to form a layer 71 mm in diameter and 6 mm in height. Then the de Nouy ring was descended on the sample so as to be positioned exactly on the surface. Oscillatory torque of 10 $\mu$N·m was applied at 1 Hz frequency and surface elastic modulus, G', was recorded as a function of time for 10 minutes. A relative increase of G' (defined as the value of G' after 10 minutes compared to its initial value) was recorded for Formulas B and G of Tables 4 and 5, above (respectively) and for the benchmark formulas E and J. In these tests, formulas B and G (20 weight % talc/ 20 weight % calcium carbonate), exhibit reduced product dry-out compared to the "benchmark" toothpastes with 40-42 weight % calcium carbonate abrasive.

**Table 5**

| Formula | Relative increase of G' |
|---|---|
| Benchmark "E" (of Table 4) | 4.7 |
| Benchmark "J" (of Table 5) | 4.4 |
| Formula "B" (of Table 4) | 3.5 |
| Formula "G" (of Table 5) | 3.1 |

Example 5

It has been found that the Talc-containing formulations of the present invention provide improved flavor release as compared to all-PCC or all-NCC formulations which do not contain talc, but which have equivalent solids loading (i.e. which have a weight percentage of PCC or NCC which is equivalent to the total weight percentage of talc and NCC/PCC present in the compositions of the present invention). The differences in oil absorption value for talc and for NCC/PCC are small (talc having an oil absorption value of approximately 30-40 grams per 100 grams of talc, and NCC and PCC having oil absorption values of approximately 6-10 grams and 10-20 grams per 100 grams of material, respectively). Without wishing to be bound by any theory, it is believed that the hydrophobic character of the talc combined with its soft, readily friable, lamellar, macrocrystalline structure accounts for increased flavor perception during toothpaste usage.

Four trained expert sensory panels were fielded comparing sensorial attributes of talc/calcium carbonate prototypes to benchmark formulas of different compositions and flavor systems. The initial study compared a prototype of 20 weight % talc/20 weight % PCC and equal flavor level to the benchmark (formula E in Table 3), and it was found that the prototype (corresponding to formula B in table 3, but with 18 weight % glycerin and a correspondingly-adjusted balance of water) provided greater flavor intensity during brushing and at several time points after expectorating. A subsequent study indicated parity sensory ratings for: a 20 weight % talc/20 weight % PCC toothpaste prototype corresponding to formula B in table 3, but with 18 weight % glycerin and a correspondingly-adjusted balance of water, and 10% reduced flavor loading compared to the respective benchmark formula (40 weight % PCC i.e. formula E in table 3); and the respective benchmark formula of 40 weight % PCC abrasives (formula E in table 3). These first two studies utilized the same flavor composition of predominantly anethole character.

A third expert panel evaluated the same 20 weight % talc/20 weight % PCC prototype but with 10% reduced flavor that was primarily of spearmint character. Again, it was found that the talc/PCC toothpaste of lower flavor loading provided no significant differences in sensory attributes compared to the 40 weight % PCC benchmark (formula E).

A final expert panel study compared a prototype (formula G of Table 5) containing 20 weight % talc/20 weight % NCC and 20 weight % reduced flavor loading to a benchmark formula (formula J of Table 5) containing 42 weight % NCC abrasives. Results showed that the talc/NCC formula of similar solids (total talc + NCC/PCC) content but greatly reduced flavor level had no significant downsides on key sensory attributes compared to the benchmark 42 weight % NCC formula.

The compositions of the present invention could therefore also represent a cost saving as compared to previous calcium carbonate-based formulations, as they allow for a reduction in the amount of flavor used without compromising sensorial aspects of the toothpaste.

**Claims**

1. An oral care composition comprising, by total weight of the composition:

   (a) 15 to 35 weight % calcium carbonate; and
   (b) from 10 to 20 weight % talc;

   wherein the total concentration of talc and calcium carbonate in the composition is from greater than 35 weight % to 42 weight %, by total weight of the composition;
   and wherein the calcium carbonate comprises natural calcium carbonate or precipitated calcium carbonate.

2. The oral care composition of claim 1, further comprising from 0.5 to 2 weight % cellulose ether thickening agent, based on the total weight of the composition.

3. The oral care composition of claim 1 or claim 2, further comprising an inorganic thickening agent.

4. The oral care composition of claim 3, wherein the inorganic thickening agent comprises thickening silica.

5. The oral care composition of claim 4, comprising from 1 to 6 weight % thickening silica, based on the total weight of the composition.

6. The oral care composition of claim 5, comprising from 1 to 2 weight % thickening silica, based on the total weight of the composition.

7. The oral care composition of any one of claims 3 to 6, wherein the inorganic thickening agent comprises a clay thickening agent.

8. The oral care composition of claim 7, comprising from 0.5 to 2 weight % clay thickening agent, based on the total weight of the composition.

9. The oral care composition of any one of claims 2 to 8, wherein the cellulose ether thickening agent comprises carboxymethylcellulose; sodium carboxymethyl cellulose; hydroxymethylcellulose; hydroxyethylcellulose; hydroxypropylcellulose; hydroxypropylmethylcellulose; or mixtures thereof.

10. The oral care composition of any one of claims 7 to 9, wherein the clay thickening agent is a smectite, bentonite, montmorillonite, hectorite or attipulgite clay.

11. The oral care composition of any preceding claim, wherein the talc comprises natural talc, synthetic talc, modified talc, or mixtures thereof.

12. The oral care composition of claim 11, wherein the talc is natural talc.

13. The oral care composition of any preceding claim, wherein the composition has a pH of from 9.2 to 10.2, and wherein the composition further comprises a buffer system, the buffer system being:

   (a) a combination of sodium silicate and tetrasodium pyrophosphate;
   (b) a combination of sodium hydroxide, sodium bicarbonate and tetrasodium pyrophosphate; or
   (c) a combination of sodium bicarbonate and sodium carbonate.

14. The oral care composition of any preceding claim, wherein the composition is a toothpaste, a tooth gel, or a combination thereof.

15. Use, in an oral care composition, of a combination of talc and calcium carbonate to improve flavor release.

**Patentansprüche**

1. Mundpflegezusammensetzung, die bezogen auf das Gesamtgewicht der Zusammensetzung umfasst:

   (a) 15 bis 35 Gew.-% Calciumcarbonat; und
   (b) von 10 bis 20 Gew.-% Talk;

   wobei die Gesamtkonzentration an Talk und Calciumcarbonat in der Zusammensetzung von mehr als 35 Gew.-% bis 42 Gew.-% bezogen auf das Gesamtgewicht der Zusammensetzung beträgt;
   und wobei das Calciumcarbonat natürliches Calciumcarbonat oder gefälltes Calciumcarbonat umfasst.

2. Mundpflegezusammensetzung nach Anspruch 1, die weiterhin von 0,5 bis 2 Gew.-% Celluloseether-Verdickungsmittel basierend auf dem Gesamtgewicht der Zusammensetzung umfasst.

3. Mundpflegezusammensetzung nach Anspruch 1 oder Anspruch 2, die weiterhin ein anorganisches Verdickungsmittel umfasst.

4. Mundpflegezusammensetzung nach Anspruch 3, wobei das anorganische Verdickungsmittel verdickendes Silica umfasst.

5. Mundpflegezusammensetzung nach Anspruch 4, die von 1 bis 6 Gew.-% verdickendes Silica basierend auf dem Gesamtgewicht der Zusammensetzung umfasst.

6. Mundpflegezusammensetzung nach Anspruch 5, die von 1 bis 2 Gew.-% verdickendes Silica basierend auf dem Gesamtgewicht der Zusammensetzung umfasst.

7. Mundpflegezusammensetzung nach einem beliebigen der Ansprüche 3 bis 6, wobei das anorganische Verdickungsmittel ein Ton-Verdickungsmittel umfasst.

8. Mundpflegezusammensetzung nach Anspruch 7, die von 0,5 bis 2 Gew.-% Ton-Verdickungsmittel basierend auf dem Gesamtgewicht der Zusammensetzung umfasst.

9. Mundpflegezusammensetzung nach einem beliebigen der Ansprüche 2 bis 8, wobei das Celluloseether-Verdickungsmittel Carboxymethylcellulose; Natriumcarboxymethylcellulose; Hydroxymethylcellulose; Hydroxyethylcellulose; Hydroxypropylcellulose; Hydroxypropylmethylcellulose; oder Gemische davon umfasst.

10. Mundpflegezusammensetzung nach einem beliebigen der Ansprüche 7 bis 9, wobei das Ton-Verdickungsmittel ein Smektit, Bentonit, Montmorillonit, Hektorit oder Attapulgit-Ton ist.

11. Mundpflegezusammensetzung nach einem beliebigen voranstehenden Anspruch, wobei der Talk natürlichen Talk, synthetischen Talk, modifizierten Talk oder Gemische davon umfasst.

12. Mundpflegezusammensetzung nach Anspruch 11, wobei der Talk natürlicher Talk ist.

13. Mundpflegezusammensetzung nach einem beliebigen voranstehenden Anspruch, wobei die Zusammensetzung einen pH von 9,2 bis 10,2 aufweist, und wobei die Zusammensetzung weiterhin ein Puffersystem umfasst, wobei das Puffersystem ist:

    (a) eine Kombination von Natriumsilikat und Tetranatriumpyrophosphat;
    (b) eine Kombination von Natriumhydroxid, Natriumbicarbonat und Tetranatriumpyrophosphat; oder
    (c) eine Kombination von Natriumbicarbonat und Natriumcarbonat.

14. Mundpflegezusammensetzung nach einem beliebigen voranstehenden Anspruch, wobei die Zusammensetzung eine Zahnpasta, ein Zahngel oder eine Kombination davon ist.

15. Verwendung in einer Mundpflegezusammensetzung einer Kombination von Talk und Calciumcarbonat, um Aromafreisetzung zu verbessern.

**Revendications**

1. Composition de soin buccal comprenant, en poids total de la composition :

    (a) 15 à 35 % en poids de carbonate de calcium ; et
    (b) de 10 à 20 % en poids de talc ;

    dans laquelle la concentration totale de talc et de carbonate de calcium dans la composition est supérieure à 35 % en poids à 42 % en poids, en poids total de la composition.
    et dans laquelle le carbonate de calcium comprend du carbonate de calcium naturel ou du carbonate de calcium précipité.

2. Composition de soin buccal selon la revendication 1, comprenant en outre de 0,5 à 2 % en poids d'agent épaississant éther de cellulose, par rapport au poids total de la composition.

3. Composition de soin buccal selon la revendication 1 ou la revendication 2, comprenant en outre un agent épaississant inorganique.

4. Composition de soin buccal selon la revendication 3, dans laquelle l'agent épaississant inorganique comprend de la silice épaississante.

5. Composition de soin buccal selon la revendication 4, comprenant de 1 à 6 % en poids de silice épaississante, par rapport au poids total de la composition.

6. Composition de soin buccal selon la revendication 5, comprenant de 1 à 2 % en poids de silice épaississante, par rapport au poids total de la composition.

7. Composition de soin buccal selon l'une quelconque des revendications 3 à 6, dans laquelle l'agent épaississant inorganique comprend un agent épaississant à base d'argile.

8. Composition de soin buccal selon la revendication 7, comprenant de 0,5 à 2 % en poids d'agent épaississant à base d'argile, par rapport au poids total de la composition.

9. Composition de soin buccal selon l'une quelconque des revendications 2 à 8, dans laquelle l'agent épaississant éther de cellulose comprend de la carboxyméthylcellulose ; de la carboxyméthylcellulose sodique ; de l'hydroxyméthylcellulose ; de l'hydroxyéthylcellulose ; de l'hydroxypropylcellulose ; de l'hydroxypropylméthylcellulose ; ou des mélanges de celles-ci.

10. Composition de soin buccal selon l'une quelconque des revendications 7 à 9, dans laquelle l'agent épaississant à base d'argile est une argile smectite, bentonite, montmorillonite, hectorite ou attipulgite.

11. Composition de soin buccal selon l'une quelconque des revendications précédentes, dans laquelle le talc comprend un talc naturel, un talc synthétique, un talc modifié ou des mélanges de ceux-ci.

12. Composition de soin buccal selon la revendication 11, dans laquelle le talc est du talc naturel.

13. Composition de soin buccal selon l'une quelconque des revendications précédentes, dans laquelle la composition a un pH de 9,2 à 10,2, et dans laquelle la composition comprend en outre un système tampon, le système tampon étant :

    (a) une combinaison de silicate de sodium et de pyrophosphate de tétrasodium ;
    (b) une combinaison d'hydroxyde de sodium, de bicarbonate de sodium et de pyrophosphate de tétrasodium ; ou
    (c) une combinaison de bicarbonate de sodium et de carbonate de sodium.

14. Composition de soin buccal selon l'une quelconque des revendications précédentes, dans laquelle la composition est une pâte dentifrice, un gel dentaire ou une combinaison de ceux-ci.

15. Utilisation, dans une composition de soin buccal, d'une combinaison de talc et de carbonate de calcium pour améliorer la libération de saveur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **STOOKEY, G. K. et al.** In vitro removal of stain with dentifrice. *Journal of Dental Research,* 11 November 1982, vol. 61, 1236-1239